(19) Europäisches Patentamt
European Patent Office
Office européen des brevets

(11) Publication number : 0 485 158 A2

# (12) EUROPEAN PATENT APPLICATION

(21) Application number : 91310205.9

(22) Date of filing : 05.11.91

(51) Int. Cl.[5] : C08B 37/00, C08F 8/00, C08G 73/04, A61K 47/48

(30) Priority : 06.11.90 US 609799
11.10.91 US 776889

(43) Date of publication of application :
13.05.92 Bulletin 92/20

(84) Designated Contracting States :
BE CH DE ES FR GB IT LI NL SE

(71) Applicant : FGN, INC.
Ventana Canyon Center, 5620 North Kolb Road
Tucson, Arizona 85715 (US)

(71) Applicant : THE UNIVERSITY OF ARIZONA
Tucson, Arizona 85721 (US)

(72) Inventor : Pamukcu, Rifat
1224 Herschel
Cincinnati, Ohio 45208 (US)
Inventor : Gross, Paul
126 West McKenzie
Stockton, California 95204 (US)
Inventor : Brendel, Klaus
3231 North Manor Drive
Tucson, Arizona 85715 (US)

(74) Representative : Baverstock, Michael George Douglas et al
BOULT, WADE & TENNANT 27 Furnival Street
London, EC4A 1PQ (GB)

## (54) Esters and amides of substituted phenyl acetic acids.

(57) Esters and amides of substituted phenyl acetic acids having the formula

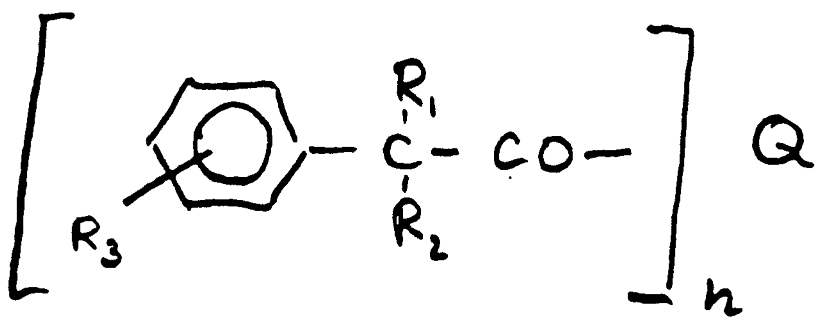

wherein Q is a deprotonated residue of a polymer or macromolecular structure having a molecular weight of at least 1000 containing at least two primary and/or secondary amino groups and/or hydroxy groups ;

n is an integer of at least 2 ;

$R_1$ and $R_2$ are independently selected from

hydrogen, halogen, alkyl, alkenyl, alkinyl or halo alkyl ;

$R_3$ is one or more substituents selected from halogen, alkyl, alkenyl or alkinyl, cycloalkyl, oxo-substituted alkylcycloalkyl, haloalkyl, alkoxy, alkenyloxy, alkinyloxy, alkylamino, alkenylamino, alkinylamino, alkysulfonyl, alkenylsulfonyl, alkinylsulfonyl, alkylsulfinyl, alkenylsulfinyl, alkinylsulfinyl, alkylsulfenyl, alkenylsulfenyl, alkinylsulfenyl, pyrrolyl, piperidinyl, benzoyl, imidazolpyridinyl, isoindolyl, oxo-substituted isoindolyl, thionylcarbonyl, phenyl, phenoxy and halo-substituted phenoxy are useful in the treatment of colonic polyps.

EP 0 485 158 A2

Jouve, 18, rue Saint-Denis, 75001 PARIS

This invention relates to compounds and compositions for use in the treatment or prevention of colonic polyps.

Each year in the United States alone, approximately 60,000 people die from colon cancer, and over 150,000 new cases of colon cancer are diagnosed. For the American population as a whole, individuals have a six percent lifetime risk of developing colon cancer, making it the second most prevalent form of cancer in the country. Colon cancer is also prevalent in Western Europe.

To date, little progress has been made in the prevention and treatment of colorectal cancer, as reflected by the lack of change in the five-year survival rate over the last few decades. The only cure for this cancer is surgery at an extremely early stage. Unfortunately, most of these cancers are discovered too late for surgical cure, because most victims do not experience symptoms until the disease is advanced.

The incidence of colon cancer increases with age, particularly after the age of 40. Since the mean ages of populations in America and Western Europe are increasing, the prevalence of colorectal cancer should increase in the future.

In view of these grim statistics, efforts in recent years have concentrated on colon cancer prevention. Colon cancer usually arises from pre-existing benign growths known as polyps. Prevention efforts have emphasized the identification and removal of colonic polyps. Polyps are identified by x-ray and/or colonoscopy, and usually removed by devices associated with the colonoscope. The increased use of colon x-rays and colonoscopies in recent years has detected clinically significant precancerous polyps in four to six times the number of individuals per year that acquire colon cancer. During the past five years alone, an estimated 3.5 to 5.5 million people in the United States have been diagnosed with adenomatous colonic polyps, and it is estimated that many more people have or are susceptible to developing this condition, but are as yet undiagnosed. In fact, there are estimates that 10-12 percent of people over the age of 40 will form clinically significant adenomatous polyps.

Removal of polyps has been accomplished either with surgery or fiber-optic endoscopic polypectomy -- procedures that are uncomfortable, costly (the cost of a single polypectomy ranges between $1,000 and $1,500 for endoscopic treatment and more for surgery), and involve a small but significant risk of colon perforation. Overall, about $2.5 billion is spent annually in the United States in colon cancer treatment and prevention.

As indicated above, each polyp carries with it a chance that it will develop into a cancer. The likelihood of cancer is diminished if a polyp is removed. However, many of these patients demonstrate a propensity for developing additional polyps in the future. They must, therefore, be monitored periodically for the rest of their lives for polyp reoccurrence.

In most cases (i.e. the cases of so-called common sporadic polyps), polyp removal will be effective to reduce the risk of cancer. In a small percentage of cases (i.e. the cases of the so-called polyposis syndromes), removal of all or part if the colon is indicated. The difference between common sporadic polyps and polyposis syndromes is dramatic. Common sporadic polyp cases are characterized by relatively few polyps, each of which can usually be removed leaving the colon intact. By contrast, polyposis syndrome cases can be characterized by many (e.g. hundreds or more) of polyps- literally covering the colon in some cases, making safe removal of the polyps impossible short of surgical removal of the colon. Because each polyp carries with it the palpable risk of cancerous development, polyposis syndrome patients invariably develop cancer if left untreated. Many of these patients have undergone a severe change in lifestyle as a result of surgery. Patients have strict dietary restrictions, and many must wear ostomy appliances to collect their intestinal wastes.

Recently, several non-steroidal anti-inflammatory drugs ("NSAIDs"), originally developed to treat arthritis, have shown effectiveness in inhibiting and eliminating polyps. Polyps virtually disappear when the patient take the drug. However, the prophylactic use of currently available NSAIDs, even in polyposis syndrome patients, is marked by severe side reactions that include gastrointestinal irritations and ulcerations. Once NSAID treatment is terminated due to such complications, the polyps return, particularly in syndrome polyposis patients.

This invention provides a novel class of compounds of formula I below that are effective in eliminating and inhibiting polyps, but are not characterized by the severe side reactions of NSAIDs:

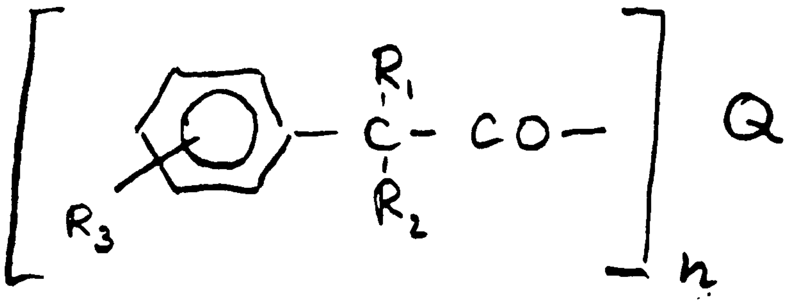

I

wherein Q is the deprotonated residue of a polymer or macromolecular structure having a molecular weight of at least 1000 containing at least two primary and/or secondary amino groups and/or hydroxy groups;

n is an integer of at least 2;

$R_1$ and $R_2$ are independently selected from hydrogen, halogen, alkyl, alkenyl, alkinyl or halo alkyl;

$R_3$ is one or more selected from

halogen, alkyl, alkenyl or alkinyl, cycloalkyl, oxo-substituted alkylcycloalkyl, haloalkyl, alkoxy, alkenyloxy, alkinyloxy, alkylamino, alkenylamino, alkinylamino, alkysulfonyl, alkenylsulfonyl, alkinylsulfonyl, alkylsulfinyl, alkenylsulfinyl, alkinylsulfinyl, alkylsulfenyl, alkenylsulfenyl, alkinylsulfenyl, pyrrolyl, piperidinyl, benzoyl, imidazolpyridinyl, isoindolyl, oxo-substituted isoindolyl, thionylcarbonyl, phenyl, phenoxy and halo-substituted phenoxy.

This invention also enables a method of treating patients with common sporadic polyps and polyposis syndrome to reduce or eliminate their polyps by administering to a patient in need of such treatment a physiologically effective amount of a compound of formula I, wherein n and $R_1$ - $R_3$ are as defined above, and Q is the deprotonated residue of a polyamino or polyhydroxy compound.

As discussed above, the present invention provides a class of compounds of formula I above, as well as a method of treating individuals with common sporadic polyps and polyposis syndromes by the administration of such a compound. Preferred compounds of the present invention are those of formula I:

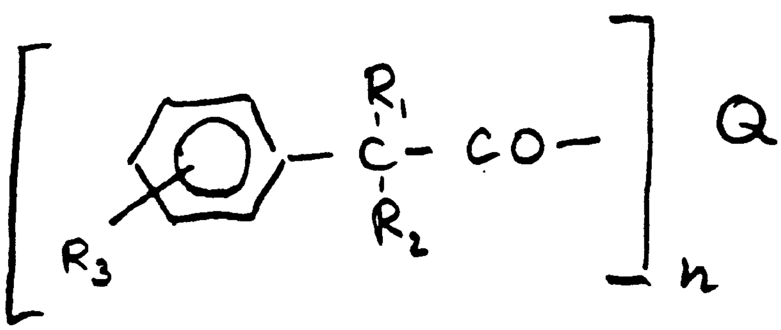

I

wherein $R_1$ is hydrogen, and $R_2$ is alkyl. The most preferred compounds are where $R_1$ is hydrogen, $R_2$ is alkyl and $R_3$ is a branched alkyl, alkenyloxy, alkenylamino, pyrroline, isoindolyl, meta- or p-phenoxy or phenalkoxy, benzoyl or thionylcarbonyl.

Examples of compounds of this invention include polyibuprofenylamidoethylcellulose, 4-polyisobutylphenyl acetate conjugate with polyvinyl alcohol, polypiroprofenyl chitosan, poly-∊-(4-piperidinophenyl)-acetyllysine, polyfeneloracyl methyl cellulose, polyaclofenacyl chitosan, polyindoprofenyl ester of polyvinyl alcohol, poly-4-pyrrolinophenylacetamidoethyl cellulose, polyfenoprofenyl ester of polyvinyl alcohol, poly-3-phenoxyphenylacetyl chitosan, polysuprofenyl chitosan, polybutibufenylamidoethyl cellulose, polyketoprofenyl chitosan, and poly-3-benzoyl phenyl acetyl ester of methyl cellulose.

As used herein, the term "halo" or "halogen" refers to chloro, bromo, fluoro and iodo groups. The term "alkyl" refers to straight or branched chains or cyclic groups, preferably having from one to four carbon atoms. The

term "alkoxy" refers to straight, branched or cyclic groups, preferably having from one to four carbon atoms. "Alkenyl" and "Alkinyl" refer to straight or branched groups, preferably having from two to five carbon atoms. The term "haloalkyl" refers to an alkyl group substituted with one or more halogens. The term "lower alkyl" refers to $C_1$-$C_5$ alkyl groups. The term "oxo-substituted alkyl cycloalkyl" refers to an alkyl group that contains an oxo-substituted cyclo-alkyl group linked to the phenyl moiety through an alkylene moiety.

As used herein, the term macromolecule, macromolecular structure, or polymer refers to molecules having at least two primary and/or secondary amino groups, and/or hydroxy groups. Examples of such amino-containing polymers or macromolecules are polyvinylamine, polyallylamine, polyethyleneimine, chitosan, polyamino acids, polyamine exchange resins (e.g. Amberlite), and polyaminoalkanes. Examples of hydroxy-containing polymers or macromolecules are polyhydroxyalkanes, polyvinylalcohols, carbohydrates (e.g. sucrose), and polyethylene glycols. The term "deprotonated residue" includes the situation where at least some, but not all, of the amino and/or hydroxy groups are deprotonated on the macromolecule or polymer.

Compounds of this invention have unexpected utility for suppression of colonic polyps in view of the startling discovery that the effects of conventional NSAID therapy on colonic polyps can be, in fact, achieved via topical exposure to the agents. This effect was discovered in a patient with familial polyposis, a disease characterized by the presence of numerous colonic polyps. In an attempt to avoid colon cancer, the patient underwent surgical excision of the colon with formation of a continent ileostomy, or Kock's pouch. By this rarely performed surgical procedure, a pouch is constructed from the terminal portion of the small intestine. A colonic bacterial environment developed within the pouch resulting in extensive adenomatous polyp formation. Polyps also developed on the stoma, an external outlet from the pouch constructed from a contiguous portion of small intestine, and in the duodenum, the beginning of the small intestine.

An NSAID, when administered in oral doses, led to the disappearance of the numerous polyps located in the pouch but not the polyps on the stoma or in the duodenum. Given an understanding of the metabolic and excretory patterns of the drug as well as of bacterial enzyme activation of the agent, these rare findings suggested that high local concentrations of the drug were responsible for the effects in the pouch. It was apparent from the lack of response of the polyps in the other locations, particularly the stomal polyps which are close to the pouch, that the effect was topical and that blood-borne or systemic delivery of the drug was ineffectual.

The topical effect is particularly surprising since the cells believed to be responsible for polyp growth and subsequent malignancy are not only epithelial cells deep within the crypts of the intestine, but may also include cells which modulate the local immunological defense mechanisms in deeper mucosal and serosal layers of the intestinal wall.

Compounds of this invention deliver active agents to the colon via the large macromolecular structure to which the active agent is conjugated. Colonic bacterial enzymes (or other colonic enzymes) cleave the active agent from the macromolecule, achieving locally high concentrations of the active agent and allowing the agent to contact the colon itself leading to inhibition of polyp proliferation.

The advantage of this treatment is that the active agent can be concentrated where it is effective, but whatever systemic levels are achieved are minimized. The systemic levels are particularly low because only passive absorption in the colon is involved. The negligible systemic levels are important in that the maintenance of chronic systemic levels of NSAIDs is complicated by a high incidence of gastric ulcers rendering them useless in a long-term prophylactic regimen.

Thus, contrary to prior approaches that relied on the high systemic levels of active agent to achieve the desired effect within the colon with the consequent gastric complications, the compounds of the present invention afford a different and safer therapeutic approach in light of the topical effect of these active agents on the colon itself.

Compounds of Formula I may also be formulated into compositions together with pharmaceutically acceptable carriers for parenteral injection, for oral administration in solid or liquid form, for rectal administration, and the like, although oral administration is most preferred.

Compositions according to the present invention for parenteral injection may comprise pharmaceutically acceptable sterile aqueous or nonaqueous solutions, suspensions or emulsions. Examples of suitable nonaqueous carriers, diluents, solvents or vehicles include propylene glycol, vegetable oils, such as olive oil, and injectable organic esters such as ethyl oleate. Such compositions may also contain adjuvants such as preserving, wetting, emulsifying and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders, troches and granules. In such solid dosage forms, the active compound is admixed with at least one inert diluent such as sucrose, lactose or starch. Such dosage forms can also comprise, as is normal practice, additional substances other

than diluents, e.g., lubricating agents such as magnesium stearate. In the case of capsules, tablets, troches and pills, the dosage forms may also comprise buffering agents. Tablets, pills and granules can additionally be prepared with enteric coatings.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve polyp-eliminating activity in accordance with the desired method of administration. The selected dosage level therefore depends upon the nature of the active compound administered, the route of administration, the desired duration of treatment, and other factors. If desired, the daily dose may be divided into multiple doses for administration, e.g. two to four times per day.

Compounds of this invention can be made by one of the five general schemes below.

SCHEME I

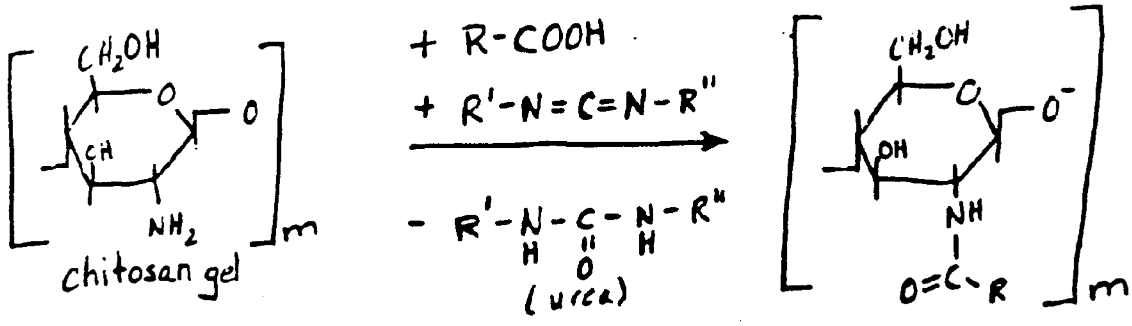


This scheme is useful for cases, where Q is water swellable polymer carrying aminogroups. The water soluble carbodiimide allows acylation in the alcoholicaqueous phase, and the water soluble by-product urea can be removed by water, from the acylated polymer. The scheme allows acylation with carboxylic acid sensitive to the conditions of acid chloride or acid anhydride formation.

A chitosan gel $(GlcN)_m$ is prepared from chitosan, according to the method of S. Hirano et al. (Carbohyd. Res. 201 (1990) pp. 145-149) where m is the number of repeating units within the chitosan molecule. The gel is stirred in 70% aqueous methanol solution, at 0°-5° C, with the R-carboxylic acid (2 equivalents per GlcN; where R is the group in the brackets in formula I minus the attached carbonyl), and with a water-soluble carbodiimide (R′-N=C=N-R″; 2 equivalents per GlcN) for three days. (R′ and R″ are cycloalkyl or alkyl and the like, containing also quaternary ammonium or sulfonate salt for solubilization of the carbodiimide.) The resulting gel is homogenized, washed well with distilled water, stirred with NaOH (1.2 equivalents per GlcN) in water (50 ml per g of chitosan) for five days. The mixture is homogenized and washed to neutrality. The gel is then dried to an amorphous powder.

SCHEME II

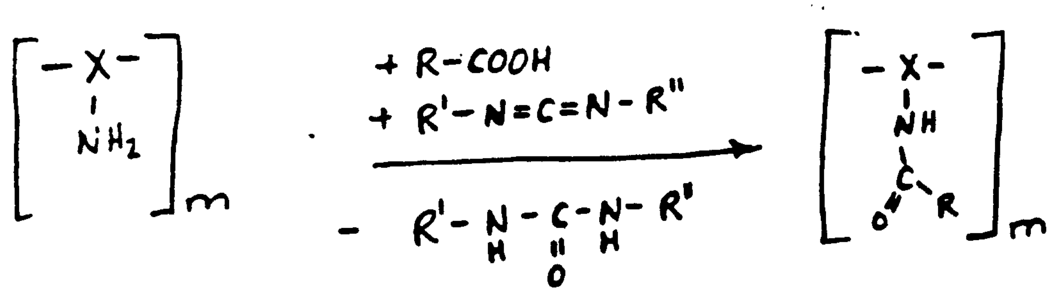

This scheme is useful for cases, where a salt between R-COOH and a polyamine Q is swellable or soluble in DMF. The carbodiimide is chosen, so that the by- product urea is soluble in dichloromethane, and therefore

removable by extraction with it. Sodium hydroxide is used to extract unreacted R-COOH, so this scheme is useful for cases where acylation is difficult and incomplete. The scheme especially allows acylation with carboxylic acids that degrade under the conditions of acid chloride or acid anhydride formation.

$(GlcN)_m$ "chitosan," polylysine or similar polyamine "X" (0.01 mol-$NH_2$ groups; where m is the number of repeating amino-containing units per molecule of polyamine) are rapidly stirred in dimethylformanide ("DMF," 30 ml) at 50°C until no further dissolution is apparent. The cooled (0°) mixture is treated with carbodiimide (R′-N=C=N-R″; 0.011 mol) with continued stirring for two days. The resultant solution or suspension is poured into ice water. The precipitate is filtered off and washed with water. It is purified by being homogenized with and filtered from (a) $CH_2Cl_2$ (2 x 50 ml); (b) 0.1 N-NaOH (2 x 50 ml); (c) 0.1N-HCl (2 x 50 ml); (d) $H_2O$ (2 x 50 ml); and (e) ether (2 x 50 ml). The resultant powder is dried.

<u>SCHEME III</u>

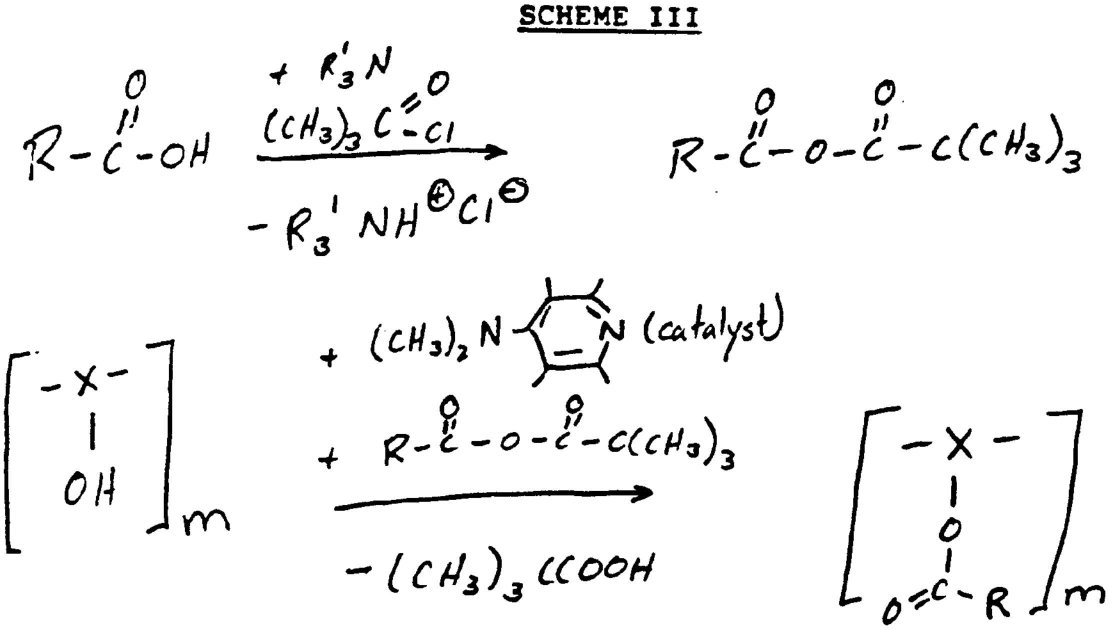


This scheme is suitable for cases were Q is a hydroxyl group-containing polymer that is soluble or swellable in dimethyl formamide. The bulky t-butyl group in pivalic acid prevents acylation by it, and dimethylamino pyridine catalyzes the difficult O-acylation. By-product pivalic acid is removable from the acylated polymer by extraction with organic solvent (e.g. toluene). The scheme is useful for carboxylic acids that are sensitive to the conditions of acylchloride synthesis.

Dry polyvinyl alcohol, methyl cellulose, or a similar swellable carbohydrate ($[X\text{-}OH]_m$; 0.01 mol-OH; where "m" is the number of repeating hydroxy-containing units in the polymeric compound) is rapidly stirred in absolute dimethyl formamide ("DMF," 50 ml) at 50°C until no further dissolution is apparent. Separately the carboxylic acid (RCOOH; 0.01 mol) is dissolved in absolute tetrahydrofuran (30 ml). At -10°C, pivaloyl chloride (0.01 mol) is added, followed by drop wise addition of a tertiary amine ($R'_3N$) (0.01 mol, e.g., triethylamine, ethyl diisopropylamine). The precipitated amine hydrochloride is filtered off. The solution is added, drop by drop, to the stirred and cooled (-10°C) polyol or carbohydrate mixture. The combined mixture is treated at -10°C with p-dimethyl amino pyridine (0.0001 mol.), and is allowed to come to room temperature and stay there for 15 hours. Toluene (100 ml) is added, with stirring. The mixture is evaporated to dryness in a rotary evaporator. The residue is homogenized in and filtered from (a) toluene (100 ml) and (b) water (2 x 100 ml). The filtercake is dried <u>in vacuo</u> at 40°C to constant weight.

## SCHEME IV

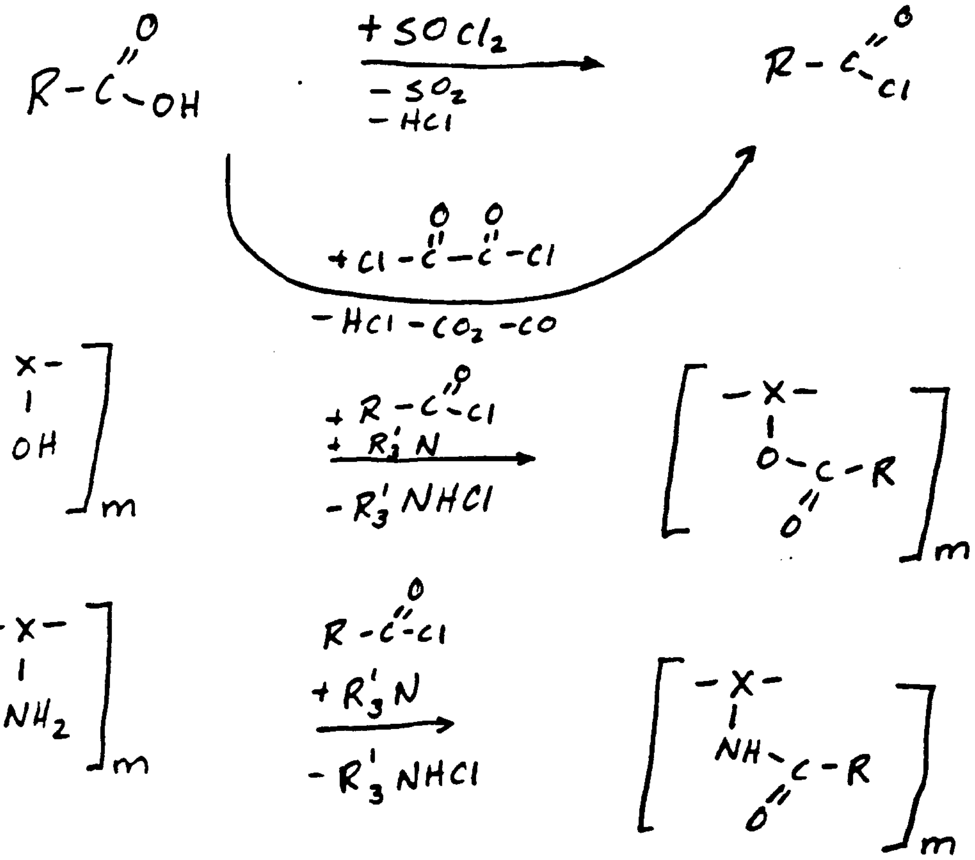


This scheme is useful for cases where Q is a polymer swellable in dimethyl formamide (DMF), and where it needs a highly reactive reagent for acylation. The scheme is suitable for carboxylic acids that form stable acid chlorides.

Carboxylic acid (R-COOH; 0.01 mol) is refluxed with thionylchloride or oxalylchloride (20 ml) until solution is complete and gas evolution ceases. Excess reagent is removed by evaporation. The residual acid chloride is diluted with tetrahydrofuran (10 ml) to give solution A.

A polyamine ($[-X-NH_2]_m$) such as chitosan, aminoethyl cellulose, polylysine (0.01 mol-$NH_2$), or a polyhydroxy compound ($[-X-OH]_m$) such as polyvinyl alcohol or a carbohydrate (e.g. methyl cellulose; 0.01 mol-OH) are heated in absolute dimethyl formamide at 50°C, until no further dissolution is apparent. Pyridine (0.01 mol) and p-dimethylaminopyridine (0.01 mol) are added. The mixture is cooled to -10°C, and solution A is added slowly with stirring. After 15 hours at room temperature, toluene (100 mol) is added, and the solution is evaporated in vacuo. The residue is homogenized with and filtered from (a) water (2 x 100 ml); (b) ether (2 x 100 ml), and is dried.

SCHEME V

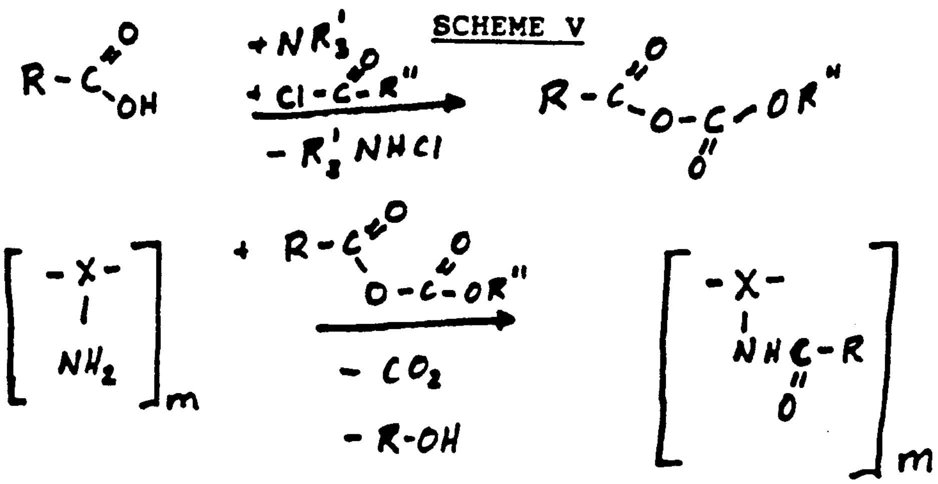


This scheme is useful, where Q is a polyamine swellable or soluble in dimethylformamide. It is especially suitable for cases where the removal of by-products such as salts, acids, or bases) from the final product is difficult, since in this case only carbon dioxide and low molecular weight alcohol are produced as by-products. The scheme is especially useful for carboxylic acids that decompose under the conditions of acid chloride synthesis.

Chitosan, amino ethyl cellulose, polylysine or a similar polyamine ([-X-$NH_2$]; 0.01 mol -$NH_2$ groups) is rapidly stirred in dimethyl formamide (30 ml) at 50°C until no further dissolution is apparent. The carboxylic acid (RCOOH; 0.01 mol) is dissolved in absolute tetrahydrofuran (30 ml). At first trialkylamine ($NR'_3$; 0.01 mol), and then alkylchlorocarbonate (Cl-COOR"; 0.01 mol, where R" is ethyl or isobutyl) is added. The precipitated trialkylammonium chloride ($R'_3NHCl$) is filtered off. The filtrate is added, with stirring, to the cold (-30°C) polyamine solution. After being stored for 15 hours at -15°C, the mixture is poured on ice (300 g), with stirring. After the ice has melted, the precipitate is filtered off, is thoroughly washed with water, and is dried.

The foregoing may be better understood from the following examples, which are presented for purposes of illustration and are not intended to limit the scope of the invention. As used in the following examples, the references to compounds such as (1), (2), (3) etc., and to substituents such as R, $R_1$, $R_2$ etc., refer to the corresponding compounds and substituents in the foregoing reaction schemes and in formula I.

## EXAMPLE 1

### Polyibuprofenyl Aminoethylcellulose

Ibuprofen (0.01 mol) is conjugated to aminoethylcellulose (0.01 mol-$NH_2$) according to Scheme IV. Specifically, thionylchloride was used to prepare the acid chloride of ibuprofen. With pyridine as the base, the procedure yields the desired compound ($R_1$= hydrogen; $R_2$= $CH_3$; $R_3$ = 4-isobutyl; Q = aminoethylcellulose; m ≧ 50; n/m ≧ 0.8; n ≧ 40).

## EXAMPLE 2

### Poly [4-isobutylphenylacetyl] Ester of Polyvinyl Alcohol

a) 4-Isobutylphenyl acetic acid

4-isobutylacetophenone (49.4 g.), sulphur (13.6 g.) and morpholine (38 ml.) are refluxed for 16 hours; concentrated hydrochloric acid (344 ml.) and glacial acetic acid (206 ml.) are added, and the mixture is refluxed for a further 7 hours. The mixture is cooled, diluted with water, and the oil that separates is isolated with ether. The ethereal solution is extracted into aqueous sodium carbonate from which the crude acid is precipitated by adding hydrochloric acid. The crude acid is again isolated with ether, and the solution is washed with water and evaporated to dryness to give a crystalline residue. The residue is crystallized from light petroleum (B.P. 40-60°C) to give 4-isobutylphenylacetic acid, M.P. 85.5-87.5°C. (Found: C. 74.1; H. 8.5 $C_{12}H_{15}O_2$ requires C. 75.0; H. 8.3%.)

b) 4-Polyisobutylphenyl Acetate Conjugate with Polyvinyl Alcohol

4-isobutylphenyl acetic acid (0.01 mol) is conjugated to polyvinyl alcohol (0.01 mol -OH) according to Scheme IV. Specifically, oxalyl chloride is used to prepare the acid chloride. With triethyl amine as the base, the procedure yields the desired compound ($R_1$, = $R_2$ = H; $R_3$ = 4-isobutyl; Q = polyvinyl alcohol; m ≧ 100; n/m ≧ 0.9; m ≧ 90).

EXAMPLE 3

Polypirprofenyl chitosan

Pirprofen (0.01 mol) is conjugated to chitosan according to Scheme I.

The procedure yields the desired product ($R_1$ = $CH_3$; $R_2$ = hydrogen; $R_3$ = m-Cl and p-N-pyrrolyl; Q = chitosan; m ≧ 40; n/m ≧ 0.7; m ≧ 28).

EXAMPLE 4

Poly [∊-(4-Piperidinophenyl)-Acetyl]polylysine

a) 4-piperidino acetophenone

A mixture of 4-fluoro-acetophenone (202 g.), piperidine (225 g.) and dimethylsulfoxide (450 ml.) is heated at a steam cone for 48 hours. After cooling, it is poured into ice water, the precipitate formed is filtered off, and recrystallized from hexane to yield the 4-piperidino-acetophenone melting at 85-86°.

b) (4-piperidinophenyl)-thioacetmorpholid

A mixture of 4-piperidino-acetophenone (45 g.), morpholine (200 ml.), sulfur (8.5 g.), and p-toluene sulfonic acid (2 g.) is refluxed for 17 hours while stirring. It is evaporated in vacuo, and the residue recrystallized from ethanol, to yield the (4-piperidinophenyl)-thioacetmorpholid of the formula

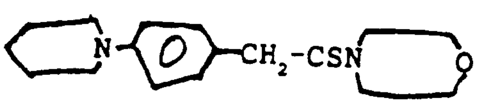

melting at 156-158°

c) 4-piperidinophenyl) - acetic acid • HCl

A mixture of (4-piperidinophenyl)-thioaceticmorpholid (42 g.) and concentrated hydrochloric acid (250 ml.) is slowly heated to reflux and refluxed for 3 hours. It is evaporated in vacuo, the residue triturated with chloroform and recrystallized from isopropanol, to yield the (4-piperidinophenyl)-acetic acid hydrochloride of the formula:

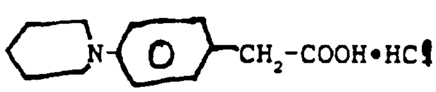

melting at 189-193°.

d) Poly (∊-(4-piperidinophenyl)-acetyl]polylysine

(4-piperidinophenyl)acetic acid is prepared in situ, in the DMF solution of poly-L-lysine with one equivalent $NET_3$. The synthesis proceeds according to Scheme II to yield the desired product ($R_1$ = $R_2$ = H; $R_3$ = p-N-piperidyl; Q = polylysine; m ≧ 40; n/m ≧ 0.8; n ≧ 32).

EXAMPLE 5

Polyfencloracyl Methyl Cellulose

This product is synthesized from fenclorac and methyl cellulose according to Scheme III. The procedure yields the desired product ($R_1$ = Cl; $R_2$ = H; $R_3$ =m-Cl and p-cyclohexyl; Q = methylcellulose; m ≧ 50; n/m ≧ 0,7; m≧ 35).

EXAMPLE 6

Polyalclofenacyl Chitosan

Alclofenac (0.01 mol) is conjugated to chitosan (0.01 mol-$NH_2$) according to Scheme V. The procedure yields the desired compound ($R_1$ = H; $R_2$ = H; $R_3$ = p-allyloxy-Cl; Q = chitosan; m ≧ 40; n/m ≧ 0.8; n ≧ 32).

EXAMPLE 7

Polyindoprofenyl Ester Of Polyvinyl Alcohol

Indoprofen (0.01 mol.) is conjugated to polyvinyl alcohol (0.01 mol-OH) according to Scheme III. Specifically, triethylamine is used as the base. The procedure yields the desired compound ($R_1$ = $CH_3$; $R_2$ = H; $R_3$ = p-(1-oxo-2-isoindolinyl); Q = polyvinyl alcohol; m ≧ 100; n/m ≧ 0.8; n ≧ 80).

EXAMPLE 8

Poly [4-Pyrrolino Phenylacetamido Ethyl] Cellulose

a) 4-pyrrolino phenyl acetic acid

A mixture of ethyl 4-amino-phenylacetate hydrochloride (10.8 g.), 1,4-dibromo-2-butene (32.4 g.), sodium bicarbonate (84 g.) and dimethylformamide (500 ml.) are refluxed for 6 hours while stirring, filtered hot, and the filtrate evaporated in vacuo. The residue is taken up in 25% aqueous sodium hydroxide (150 ml.), the mixtures refluxed for one hour, cooled and washed with diethyl ether. It is adjusted to pH 5 with hydrochloric acid, extracted with diethyl ether, the extract dried, filtered and evaporated, to yield the 4-pyrrolino-phenylacetic acid of the formula.

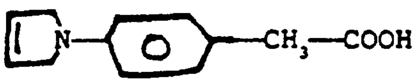

melting at 162-165°.

b) poly-4-pyrrolino phenylacetamido ethyl cellulose

4-pyrrolino phenyl acetic acid is conjugated to amino ethyl cellulose according to Scheme V. The procedure yields the desired product ($R_1$ = $R_2$ = H; $R_3$ = p-N-pyrrolinyl; Q = ethyl cellulose; m ≧ 50; n/m ≧ 0.9; n ≧ 45).

EXAMPLE 9

Polyfenoprofenyl Ester Of Polyvinyl Alcohol

Fenoprofen (0.01 mol-OH) is conjugated to polyvinyl alcohol (0.01 mol-OH) according to Scheme IV. Specifically, oxalyl chloride is used to prepare the acid chloride of fenoprofen. Pyridine is the base. The procedure yields the desired compound ($R_1$ = $CH_3$; $R_2$ = H; $R_3$ = meta-phenoxy; Q = polyvinyl alcohol; m ≧ 50; n/m ≧ 0.8; n ≧ 40).

EXAMPLE 10

Poly [3-phenoxyphenylacetyl] Chitosan

a) 2-(3-Phenoxyphenyl) Acetic Acid

To morpholine (26 ml.) is added m-phenoxyacetophenone (42.4 g.) and sulfur (9.6 g.). The reaction mixture is refluxed with stirring for 20 hours. To the reaction mixture is then added 15 percent aqueous potassium hydroxide (700 ml.) and a small amount of ethyl alcohol. The reaction mixture is refluxed with stirring for an additional 20 hours. The solvent is distilled out. The remaining reaction mixture is filtered while hot, partially cooled with ice, and acidified with concentrated hydrochloric acid, whereupon an oily precipitate forms and then crystallized. The crystalline precipate is filtered, washed several times with water, and dried to yield 45.9 g. of crude product as a yellow-orange solid. The crude product is suspended in boiling hexane, and ethyl acetate is added until the product goes into solution. The solution is then treated with carbon, filtered and cooled, to yield 22.7 g. of white flakes of 2-(3-phenoxyphenyl) acetic acid, M.P. 84-86° C.; pK'a=6.9.

Analysis-Calc for $C_{14}H_{12}O_3$ (percent): C, 73.66; H, 5.30. Found (percent); C, 73.85; H, 5.35.

b) Poly [3-phenoxyphenylacetyl] Chitosan

This product is synthesized from 3-phenoxyphenyl acetic acid (0.01 mol) and chitosan (0.01 mol-$NH_2$) according to Scheme II. The procedure yields the desired product ($R_1 = R_2 = H$; $R_3$ = meta-phenoxy; Q = chitosan; $m \geqq 50$; $n/m \geqq 0.9$; $n \geqq 45$).

EXAMPLE 11

Polysuprofenyl Chitosan

Suprofen (0.02 mol) is conjugated to chitosan gel (0.01 mol-$NH_2$) according to Scheme I. The procedure yields the desired compound ($R_1 = CH_3$; $R_2 = H$; $R_3$ = p-2-thienylcarbonyl; Q = chitosan; $m \geqq 50$; n/m 0.9; $n \geqq 45$).

EXAMPLE 12

Poly[butibufenyl Amidoethyl] Cellulose

Butibufen (0.01 mol) is conjugated to aminoethylcellulose according to Scheme IV. Specifically, thionylchloride is used to prepare the acid chloride of butibufen with pyrridine as the base. The procedure yields the desired compound ($R_1$ = ethyl; $R_2 = H$; $R_3$ = p-isobutyl; Q = aminoethylcellulose; $m \geqq 50$; $n/m \geqq 0.8$; $n \geqq 40$).

EXAMPLE 13

Polyketoprofenyl Chitosan

Ketoprofen (0.01 mol) is conjugated to chitosan according to Scheme II. The procedure yields the desired product ($R_1 = CH_3$; $R_2 = H$; $R_3$ = meta-benzoyl; Q = chitosan $m \geqq 50$; $n/m \geqq 0.8$; $n \geqq 40$).

EXAMPLE 14

Poly-[3-Benzoylphenyl Acetyl] Ester Of Methyl Cellulose

a) 3-Benzoyl Phenylacetic Acid

A mixture of (3-benzoylphenyl)acetonitrile (30 g.), concentrated sulphuric acid (60 cc.) and water (60 cc.) is heated under reflux under nitrogen for 10 minutes. Water (180 cc.) is added, and a product crystallizes, which is separated by filtration and washed with water (100 cc.). There is obtained 24 g. of a product, which is dissolved in diethyl ether (150 cc). The ethereal solution is extracted with N sodium hydroxide (200 cc.), and the alkaline solution is treated with decolorizing charcoal (1 g.), and then acidified with concentrated hydrochloric

acid (25 cc.). An oil separates out, which is extracted with methylene chloride (450 cc.), washed with water (100 cc.) and dried over anhydrous sodium sulphate. The product is concentrated to dryness under reduced pressure (20.mm. Hg) to give a white crystalline residue (18 g.), M.P. 114-115° C., which is recrystallized from a mixture of benzene (120 cc) and petroleum ether (130 cc.) to yield 3-benzoylphenylacetic acid (17.3 g.), M.P. 114-115° C.

(3-benzoylphenyl) acetonitrile employed as the starting material is prepared as follows:

3-bromomethylbenzophenone (160 g.) is dissolved in dioxane (300 cc.), and a solution of sodium cyanide (125 g.) in water (300 cc.) is added. The mixture is heated under reflux for three hours, and then treated with charcoal (10 g.) and extracted with methylene chloride (800 cc.). The methylene chloride solution is dried over anhydrous sodium sulphate and concentrated to dryness under reduced pressure (20 mm. Hg) to give a brown oil (119 g.), which is dissolved in methylene chloride (300 cc.) and chromatographed through alumina (450 g.) Elution is effected with methylene chloride, and there is collected a fraction of 4 liters, which is concentrated to dryness under reduced pressure (20 mm. Hg) to yield (3-benzoylphenyl) acetonitrile (109 g.) in the form of an oil.

3-bromomethylbenzophenone is prepared by dissolving 3-methylbenzophenone (95 g.) in ethylene bromide (200 cc.), heating the solution under reflux, and adding a solution of bromine (79 g.) in ethylene bromide (60 cc.) over a period of 3 hours in the presence of ultra-violet light. Heating under reflux is continued for 30 minutes, and the product is concentrated to dryness under reduced pressure (20 mm. Hg) to give 3-bromomethylbenzophenone in the form of an oil in quantitative yield.

3-methylbenzophenone is prepared in accordance with E. Ador and A.A. Rilliet, Ber., 12, 2298 (1879).

b) Poly-3-Benzoylphenyl Acetyl Ester Of Methyl Cellulose

The ester is prepared from 3-benzoylacetic acid (0.01 mol) and methyl cellulose (0.01 mol-OH), according to Scheme IV, with oxalylchloride for the preparation of the acid chloride, and triethylamine for the preparation of the ester. The desired product is obtained ($R_1$ = $R_2$ = H; $R_3$ = m-benzoyl; Q = methylcellulose; $m \geqq 40$; $n/m \geqq 0.7$; $n \geqq 35$).

EXAMPLE 15

Polyflurbiprofenyl Chitosan

Flurbiprofen (0.01 mol.) is conjugated to chitosan (0.01 mol-$HN_2$) according to Scheme IV. Specifically, oxalylchloride is used to prepare the acid chloride of flurbiprofen. Triethylamine is the base. The procedure yields the desired compound $R_1$ = hydrogen; $R_2$ = methyl; $R_3$ = m-Cl and ρ-phenyl; Q = chitosan; $m \geqq 50$; $n/m \geqq 0.2$; $n \geqq 10$.)

EXAMPLE 16

Polyfenclofenacylpolylysine

Fenclofenac (0.01 mol.) is conjugated to polylysine (0.01 mol-$\epsilon NH_2$) according to Scheme IV. Specifically, thionylchloride is used to prepare the acid chloride of fenclofenac. Triethylamine is the base. The procedure yields the desired compound ($R_1$ = hydrogen; $R_2$ = hydrogen; $R_3$ = o-(2,4 dichlorophenoxy); Q = polylysine; $m > 50$; $n/m > 0.2$; $n > 20$.)

EXAMPLE 17

Polyxenbucinyl Ester Of Polyvinyl Alcohol

Xenbucin is conjugated to polyvinyl alcohol according to Scheme III. Specifically, triethylamine is used as the base. The procedure yields the desired compound ($R_1$ = hydrogen; $R_2$ = ethyl; $R_3$ = phenyl; Q = polyvinyl alcohol; $m \geqq 100$; $n/m \geqq 0.2$; $n \geqq 20$.)

EXAMPLE 18

Polyloxoprofenyl Chitosan

Loxoprofen is conjugated to chitosan (0.01 mol-$NH_2$) according to Scheme V. The procedure yields the desired compound ($R_1$ = hydrogen; $R_2$ = methyl; $R_3$ = ρ-(2-oxocyclopentyl methylene; Q = chitosan; m > 50; n/m > 0.2; n > 10.)

EXAMPLE 19

Polymiroprofenylpolylysine

Miroprofen (0.01 mol.) is conjugated to polylysine (0.01 mol-$NH_2$) according to Scheme IV. Specifically, thionylchloride is used to prepare the acid chloride of miroprofen. Triethylamine is the base. The procedure yields the desired compound ($R_1$ = hydrogen; $R_2$ = methyl; $R_3$ = ρ-imidazol[1,2-a]pyridinyl; Q = polylysine; m > 50; n/m > 0.2; n > 10.)

EXAMPLE 20

Polyflurbiprofenyl Polyethyleneimine

a) Flurbiprofen acid chloride

Thionyl chloride 1.64 ml (0.023 mol) was added to a suspended solution of 5 g (0.02 mol) flurbiprofen in 30 ml of toluene. The reaction mixture was refluxed under nitrogen for 60 minutes. The solvent was removed in vacuo to give flurbiprofen acid chloride as a grey oil. IR (neat) 2938, 2987, 3034, 3061, 1797, 1779, 1582, 1484, 1455, 1450 $cm^{-1}$.

b) Polyflurbiprofenyl polyethyleneimine

Flurbiprofen acid chloride 2.98 g (0.011 mol) was dissolved in 25 ml pyridine. To this stirred solution was added 0.98 g (0.54 mmol) of polyethyleneimine (purity 99% and average molecular weight 1800). The reaction mixture was refluxed for one hour. Concentration in vacuo gave a viscous residue, which was washed with 100 ml of concentrated sodium bicarbonate solution. The aqueous part was decanted, and the residue oil was washed with five 200 ml portions of water to remove pyridine. The product was dried over phosphorus pentoxide ($P_2O_5$) in a vacuum desiccator at room temperature for 12 days. It gave 3.4 g of polyflurbiprofenyl polyethyleneimine as a solid.

IR (film) 3432, 3304, 2951, 2857, 1721, 1667, 1601, 1583, 1521, 1490, 1450, 1375, 1336, 1163 $cm^{-1}$.

Elementary analysis calculated for $C_{36}H_{37}N_3O_2F_2$, FW 581;

% Theory: C, 67.66; H, 5.79; N, 6.57; F, 5.95. % Found : C, 67.49; H, 6.05; N, 6.58; F, 5.98.

The procedure yields the desired compound $R_1$ = hydrogen; $R_2$ = methyl; $R_3$ = m-F and p-phenyl; Q = polyethyleneimine; n/m = 0.66.

EXAMPLE 21

Polyflurbiprofen Ester Of Polyvinyl Alcohol

Flurbiprofen acid chloride (0.02 mol, 5.24 g) was prepared by the procedure of Example 20a and dissolved in 20 ml pyridine. To this solution was added 1.75 g (0.035 mmol) of polyvinyl alcohol ("PVA"; average molecular weight 50,000). The reaction mixture was slowly heated until all PVA dissolved and then refluxed for one hour. The clear solution was concentrated in vacuo then washed with 100 ml of concentrated sodium bicarbonate solution. The aqueous part was decanted and the residue oil was washed with five 200 ml portions of water to remove pyridine and unreacted PVA. The product was dried over phosphorus pentoxide ($P_2O_5$) in a vacuum desiccator at room temperature for 12 days. It gave 6.04 g of the polyflurbiprofen ester of polyvinyl alcohol as a solid.

IR (film) 3432, 2977, 2925, 1732, 1624, 1583, 1560, 1818, 1485, 1415 $cm^{-1}$.

Elemental analysis calculated for $C_{19}H_{19}O_3F \bullet \frac{1}{2} H_2O$; FW 320;

% Theory: C, 71.25; H, 6.25; F, 5.93. % Found : C, 70.99; H, 5.72; F, 5.45.

The procedure yields the desired compound $R_1$ = hydrogen; $R_2$ = methyl; $R_3$ = m-F and p-phenyl; Q = polyvinyl alcohol;
n/m = 0.50.

EXAMPLE 22

Polyindoprofen Ester Of Polyvinyl Alcohol

a) Indoprofen acid chloride

Thionyl chloride (1.43 ml, 0.019 mol) was added to a suspended solution of 5 g (0.018 mol) of indoprofen in 30 ml toluene. The mixture was refluxed under nitrogen for 60 minutes. Toluene was removed in vacuo. The yellow oil was purified by crystallization in a solvent mixture of toluene/petroleum ether to give 4.14 g of indoprofen acid chloride as a white powder: mp 125-128°C. IR(Nujor Mull) 2948, 2912, 1770, 1623, 1460, 1377 $cm^{-1}$.

b) Polyindoprofen ester of polyvinyl alcohol

Indoprofen acid chloride 4.14 g (0.014 mol) was dissolved in 20 ml pyridine. To this solution was added 1.22 g (0.024 mol) of polyvinyl alcohol ("PVA", average molecular weight of 50,000). The reaction mixture was slowly heated until all PVA dissolved and then refluxed for 90 min. The clear solution was concentrated in vacuo then washed with 100 ml of concentrated sodium bicarbonate solution. The aqueous part was decanted and the residual oil was washed with five 200 ml portions of water to remove pyridine and unreacted PVA. The product was dried over phosphorus pentoxide ($P_2O_5$) in a vacuum desiccator at room temperature for 12 days. It gave 5.25 g of polyindoprofen ester of polyvinyl alcohol as a yellow solid.

IR (film) 3442, 3061, 2927, 1731, 1689, 1518, 1386, 1307 $cm^{-1}$.

Elemental analysis calculated for $C_{21}H_{21}NO_4 \bullet \frac{1}{2} H_2O$, FW 378; % Theory: C, 66.84; H, 5.97; N, 3.71. % Found : C, 66.34; H, 6.23; N, 4.56.

The procedure yields the desired compound $R_1$ = methyl; $R_2$ = hydrogen; $R_3$ = p-(1-oxo-2-isoindolinyl); Q = polyvinylalcohol; n/m = 0.50.

EXAMPLE 23

Polysuprofen Ester Of Polyvinyl Alcohol

a) Suprofen acid chloride

Thionyl chloride (1.55 ml, 0.021 mol) was added to a suspended solution of 5 g (0.019 mol) of suprofen in 30 ml toluene. The mixture was refluxed under nitrogen for 90 minutes. Toluene was removed in vacuo to give suprofen acid chloride as a yellow solid; IR(Nujor Mull) 2948, 2912, 1779, 1623, 1460, 1377, 477 $cm^{-1}$.

b) Polysuprofen ester of polyvinyl alcohol

Suprofen acid chloride (5.43 g.) was dissolved in 20 ml pyridine. To this solution was added 1.66 g (0.033 mol) of polyvinyl alcohol ("PVA", average molecular weight of 50,000). The reaction mixture was slowly heated until all PVA dissolved and then refluxed for 90 min. The clear solution was concentrated in vacuo then washed with 100 ml of concentrated sodium bicarbonate solution. The aqueous part was decanted, and the residual oil was washed with five 200 ml portions of water to remove pyridine and unreacted PVA. The product was dried over phosphorus pentoxide ($P_2O_5$) in a vacuum desiccator at room temperature for 12 days. It gave 5.27 g of polysuprofen ester of polyvinyl alcohol as a solid.

IR (film) 3407, 3061, 2928, 1737, 1729, 1624, 1582, 1484, 1450, 1331 $cm^{-1}$.

Elemental analysis calculated for $C_{18}H_{18}O_4S$, FW 330; % Theory: C, 65.64; H, 5.45; S, 9.69. % Found : C, 64.81; H, 5.47; S, 9.10.

The procedure yields the desired compound $R_1$ = methyl; $R_2$ = hydrogen; $R_3$ = p-2-thienylcarbonyl; Q = polyvinylalcohol; n/m = 0.50.

EXAMPLE 24

Polysuprofenyl Polyethyleneimine

a) Suprofen acid chloride

Thionyl chloride 1.55 ml (0.021 mol) was added to a suspended solution of 5 g (0.019 mol) suprofen in 30 ml of toluene. The reaction mixture was refluxed under nitrogen for 60 minutes. The solvent was removed in vacuo to give suprofen acid chloride as a powder. IR (Nujor Mull) 2948, 2912, 1779, 1623, 1460, 1377, 477 $cm^{-1}$.

b) Polysuprofenyl polyethyleneimine

Polyethyleneimine (purity 99% and average molecular weight 1800) 1.011 g was dissolved in 30 ml of pyridine. To this stirred solution was added 3.27 g (0.12 mol) of suprofen acid chloride. The reaction mixture was refluxed for one hour. Concentration in vacuo gave a viscous residue, which was washed with 200 ml of 1 N sodium hydroxide solution. The aqueous part was decanted, and the residue oil was washed with five 200 ml portions of water to remove pyridine. The product was dried over phosphorus pentoxide ($P_2O_5$) in a vacuum desiccator at room temperature for 12 days. It gave 1.5 g of polysuprofenyl polyethyleneimine as a solid.

IR (film) 2971, 2870, 1724, 1640, 1600, 1460, 1381, 1295 $cm^{-1}$.

Elemental analysis calculated for $C_{16}H_{15}NO_2S{\bullet}H_2O$; FW 346;
% Theory: C, 63.36; H, 5.61; N, 4.62; S, 10.56 % Found : C, 63.25; H, 5.42; N, 4.78; S, 9.41.

The procedure yields the desired compound $R_1$ = methyl; $R_2$ = hydrogen; $R_3$ = p-2-thienylcarbonyl; Q = polyethyleneimine; n/m = 1.0.

EXAMPLE 25

Polyindoprofenyl Polyethyleneimine

a) Indoprofen acid chloride

Thionyl chloride 1.51 ml (0.021 mol) was added to a suspended solution of 5.3 g (0.019 mol) indoprofen in 30 ml of toluene. The reaction mixture was refluxed under nitrogen for 60 minutes. The solvent was removed in vacuo to give indoprofen acid chloride as a white powder; mp 128°C. IR (Nujor Mull) 2964, 2915, 1775, 1666, 1519, 1461, 1457, 1377 $cm^{-1}$.

b) Polyindoprofenyl polyethyleneimine

Polyethyleneimine (purity 99% and average molecular weight 1800) 1.44 g was dissolved in 30 ml of pyridine. To this stirred solution was added 5.0 g (0.017 mol) of indoprofen acid chloride. The reaction mixture was refluxed for two hours. Concentration in vacuo gave a viscous residue, which was washed with 200 ml of 1 N sodium hydroxide solution. The aqueous part was decanted, and the residue oil was washed with five 200 ml portions of water to remove pyridine. The product was dried over phosphorus pentoxide ($P_2O_5$) in a vacuum desiccator at room temperature for 12 days. It gave 6.09 g of polyindoprofenyl polyethyleneimine as a yellow solid.

IR (film) 3456, 3314, 2971, 2870, 1691, 1645, 1608, 1516, 1458, 1381, 1304, 1223 $cm^{-1}$.

Elemental analysis calculated for $C_{40}H_{41}N_5O_5{\bullet}1.5\ H_2O$; FW 682. % Theory: C, 70.38; H, 6.30; N, 10.26. % Found : C, 70.15; H, 6.03; N, 9.66.

The procedure yields the desired compound $R_1$ = methyl; $R_2$ = hydrogen; $R_3$ = p-(1-oxo-2-isoindolinyl); Q = polyethyleneimine; n/m = 0.5.

EXAMPLE 26

Polyketoprofenyl Polyethyleneimine

a) Ketoprofen acid chloride

Thionyl chloride 2.0 ml was added to a suspended solution of 5.9 g (0.023 mol) ketoprofen in 50 ml of

toluene. The reaction mixture was refluxed under nitrogen for 60 minutes. The solvent was removed in vacuo to give ketoprofen acid chloride as a yellow liquid; IR (neat) 3009, 2927, 1784, 1661, 1598, 1448, 1461, 1381, 1281, 958, 719 $cm^{-1}$.

b) Polyketoprofenyl polyethyleneimine

Polyethyleneimine (purity 99% and average molecular weight 1800; 1.89 g) was dissolved in 50 ml of pyridine. To this stirred solution was added the solution of 6.0 g (0.022 mol) of ketoprofen acid chloride. The reaction mixture was refluxed for one hour. Concentration in vacuo gave a viscous residue, which was washed with 200 ml of 1 N sodium hydroxide solution. The aqueous part was decanted, and the residue oil was washed with five 200 ml portions of water to remove pyridine. The product was dried over phosphorus pentoxide ($P_2O_5$) in a vacuum desiccator at room temperature for 12 days. It gave 2.0 g of polyketoprofenyl polyethyleneimine as a solid.

IR (Nujor mull) 3301, 2954, 2927, 1654, 1595, 1460, 1377, 1282, 721, 703 $cm^{-1}$.

Elemental analysis calculated for $C_{38}H_{39}N_3O_4{\bullet}H_2O$; FW 619. % Theory: C, 73.66; H, 6.62; N, 6.78. % Found : C, 73.86; H, 6.23; N, 6.62.

The procedure yields the desired compound $R_1$ = methyl; $R_2$ = hydrogen; $R_3$ = meta-benzoyl; Q = polyethyleneimine; n/m = 0.666.

It will be understood that various changes and modifications can be made in the details of procedure, formulation and use without departing from the spirit of the invention, especially as defined in the following claims.

## Claims

1. A compound of the formula:

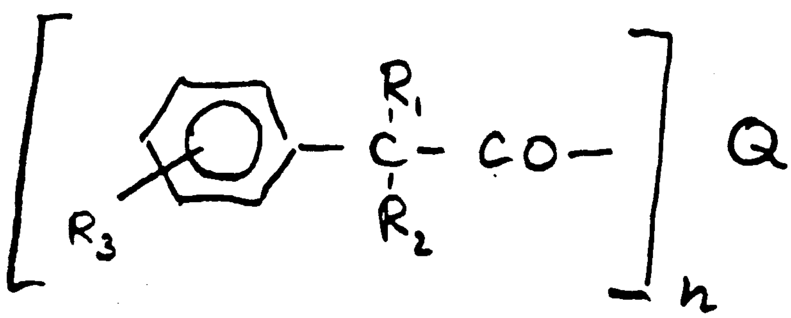

wherein Q is a deprotonated residue of a polymer or macromolecular structure having a molecular weight of at least 1000 containing at least two primary and/or secondary amino groups and/or hydroxy groups;

n is an integer of at least 2;

$R_1$ and $R_2$ are independently selected from hydrogen, halogen, alkyl, alkenyl, alkinyl or halo alkyl;

$R_3$ is one or more substituents selected from

halogen, alkyl, alkenyl or alkinyl, cycloalkyl, oxo-substituted alkylcycloalkyl, haloalkyl, alkoxy, alkenyloxy, alkinyloxy, alkylamino, alkenylamino, alkinylamino, alkysulfonyl, alkenylsulfonyl, alkinylsulfonyl, alkylsulfinyl, alkenylsulfinyl, alkinylsulfinyl, alkylsulfenyl, alkenylsulfenyl, alkinylsulfenyl, pyrrolyl, piperidinyl, benzoyl, imidazolpyridinyl, isoindolyl, oxo-substituted isoindolyl, thionylcarbonyl, phenyl, phenoxy and halo-substituted phenoxy.

2. A compound as claimed in claim 1 wherein $R_1$ is hydrogen and $R_2$ is lower alkyl.

3. A compound as claimed in claim 2 wherein $R_3$ is branched alkyl.

4. A compound as claimed in claim 2 wherein $R_3$ is alkenyloxy.

5. A compound as claimed in claim 2 wherein $R_3$ is alkenylamino.

**6.** A compound as claimed in claim 2 wherein $R_3$ is pyrroline.

**7.** A compound as claimed in claim 2 wherein $R_3$ is isoindolyl.

**8.** A compound as claimed in claim 2 wherein $R_3$ is meta or ρ-phenoxy.

**9.** A compound as claimed in claim 2 wherein $R_3$ is benzoyl or thionylcarbonyl.

**10.** A pharmaceutical preparation or composition comprising a compound as claimed in any one of the preceding claims and a pharmaceutically acceptable carrier.

**11.** Use of a compound as claimed in any one of claims 1 to 9 in the manufacture of a pharmaceutical preparation or composition for the treatment or prevention of colonic polyps.

**Claims for the following Contracting State : ES**

**1.** A method of preparing a compound for treatment of colonic polyps by conjugating a compound of formula I:

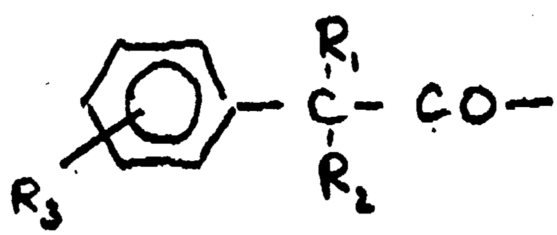

I

wherein $R_1$ and $R_2$ are independently selected from the group consisting of hydrogen, halogen, alkyl, alkenyl, alkinyl, or halo alkyl; and

$R_3$ is one or more substituents selected from the group consisting of halogen, alkyl, alkenyl or alkinyl, cycloalkyl, oxo-substituted alkylcycloalkyl, haloalkyl, alkoxy, alkenyloxy, alkinyloxy, alkylamino, alkenylamino, alkinylamino, alkysulfonyl, alkenylsulfonyl, alkinylsulfonyl, alkylsulfinyl, alkenylsulfinyl, alkinylsulfinyl, alkylsulfenyl, alkenylsulfenyl, alkinylsulfenyl, pyrrolyl, piperidinyl, benzoyl, imidazolpyridinyl, isoindolyl, oxo-substituted isoindolyl, thionylcarbonyl, phenyl, phenoxy and halo-substituted phenoxy;

with a deprotonated residue of a polymer or macromolecular structure having a molecular weight of at least 1000 containing at least two primary and/or secondary amino groups and/or hydroxy groups, wherein at least two moles of the compound of formula I are conjugated to each mole of said polymer or macromolecular structure.

**2.** A method according to claim 1 wherein $R_1$ is hydrogen and $R_2$ is lower alkyl.

**3.** A method according to claim 2 wherein $R_3$ is branched alkyl.

**4.** A method according to claim 2 wherein $R_3$ is alkenyloxy.

**5.** A method according to claim 2 wherein $R_3$ is alkenylamino.

**6.** A method according to claim 2 wherein $R_3$ is pyrroline.

**7.** A method according to claim 2 wherein $R_3$ is isoindolyl.

**8.** A method according to claim 2 wherein $R_3$ is meta or ρ-phenoxy.

9. A method according to claim 2 wherein $R_3$ is benzoyl or thionylcarbonyl.